# FASCICULE DE BREVET EUROPEEN

(11) **EP 1 440 706 B1**
(45) Date de publication et mention de la délivrance du brevet: **02.12.2009**
(21) Numéro de dépôt: 04290197.5
(22) Date de dépôt: 26.01.2004
(51) Int. Cl.: A61M 25/10

(54) **Dispositif de gonflage de ballonnet**
Aufblaseinrichtung für Ballonkatheter
Inflating device for balloon catheter

(30) Priorité: 27.01.2003 FR 0300868
(43) Date de publication de la demande: 28.07.2004
(73) Titulaire: SEDAT, 69540 Irigny (Rhône) (FR)
(72) Inventeur: Denolly, Pascal, 38200 Jardin (FR)
(74) Mandataire: Blot, Philippe Robert Emile

(56) Documents cités:
- EP-A- 0 565 045
- US-A- 4 832 692
- US-A- 5 147 300
- US-A- 6 106 496
- US-A- 6 110 151

## Description

La présente invention concerne un dispositif de gonflage de ballonnet, selon le préambule de la revendication 1.

Ces dispositifs de gonflage sont utilisés afin d'injecter un fluide dans un ballonnet préalablement disposé à l'état contracté à l'intérieur d'une artère ou d'une veine d'un malade. De tels dispositifs sont décrits par exemple dans le document US-A-5,147,300.

Ces dispositifs sont en particulier utilisés pour les angioplasties transluminales percutanées, afin notamment de dilater l'artère ou la veine dans laquelle le ballonnet est disposé.

Les dispositifs de gonflage doivent être capables de fournir une pression élevée, de l'ordre de 30 bars. Sous l'action une telle pression, le piston est soumis à une force axiale importante, qui, en l'absence de pression manuelle sur l'extrémité libre du piston, doit être contenue par la ou les demi-noix coopérant avec le filetage.

En outre, notamment dans le dispositif à une seule demi-noix décrit dans le brevet américain précité, l'organe de commande de cette demi-noix est formé par une tige dont l'extrémité est déformée en forme de manivelle. Cette extrémité est engagée dans une lumière de la demi-noix afin d'assurer le déplacement latéral de celle-ci, par déplacement axial de la tige le long du corps de seringue.

Du fait de la forte pression régnant à l'intérieur de la seringue, laquelle exerce une force élevée de poussée axiale sur le piston, la libération de la demi-noix nécessite un effort important de sorte que la tige de commande a tendance à se déformer sans assurer de manière satisfaisante sa fonction de déplacement de la demi-noix. Aussi, il est nécessaire de réaliser cet organe de commande dans un matériau extrêmement rigide, ce qui augmente le coût de fabrication du dispositif.

De plus, la tige de commande de ce dispositif est déplacée par action d'un des pouces du praticien sur un mécanisme d'entraînement en translation de la tige. Cependant, la libération de la demi-noix s'avère souvent difficile avec la sollicitation du seul pouce. En d'autres termes, l'ergonomie du dispositif n'est pas satisfaisante.

Un autre dispositif, avec un organe de commande rotatif est décrit dans le document EP.0.565.045.

L'invention a pour but de fournir un dispositif de gonflage de ballonnet, qui présente une meilleure ergonomie de manipulation permettant la mise en prise et la libération du piston de manière fiable et aisée, tout en permettant l'utilisation de matériaux communs et peu onéreux.

A cet effet, l'invention a pour objet un dispositif de gonflage de ballonnet selon la revendication 1. .

D'autres caractéristiques de ce dispositif, prises isolément ou selon toutes les combinaisons techniquement possibles figurent dans les revendications dépendantes.

L'invention sera mieux comprise à la lecture de la description qui va suivre, donnée uniquement à titre d'exemple et fait en se référant aux dessins sur lesquels :
- les figures 1A et 1B sont des vues en perspective d'un dispositif suivant l'invention, manipulé de façon à respectivement permettre le coulissement du piston de seringue et permettre le vissage/dévissage de ce piston ;
- la figure 2 est une vue en perspective et en coupe longitudinale selon un plan médian du dispositif de la figure 1A ;
- Les figures 3A et 3B sont des vues planes en coupe selon le même plan que la coupe de la figure 2, du dispositif respectivement des figures 1A et 1B;
- Les figures 4A et 4B sont des figures à plus grande échelle de détails cerclés respectivement IVA et IVB sur les figures 3A et 3B ;
- La figure 5 est une vue en perspective du dispositif de la figure 2, les organes de commande du déplacement des demi-noix n'étant pas représentés ;
- La figure 6 est une vue en perspective du dispositif de la figure 1, pourvu d'un manomètre ; et
- La figure 7 est une vue en perspective d'une variante du dispositif selon l'invention.

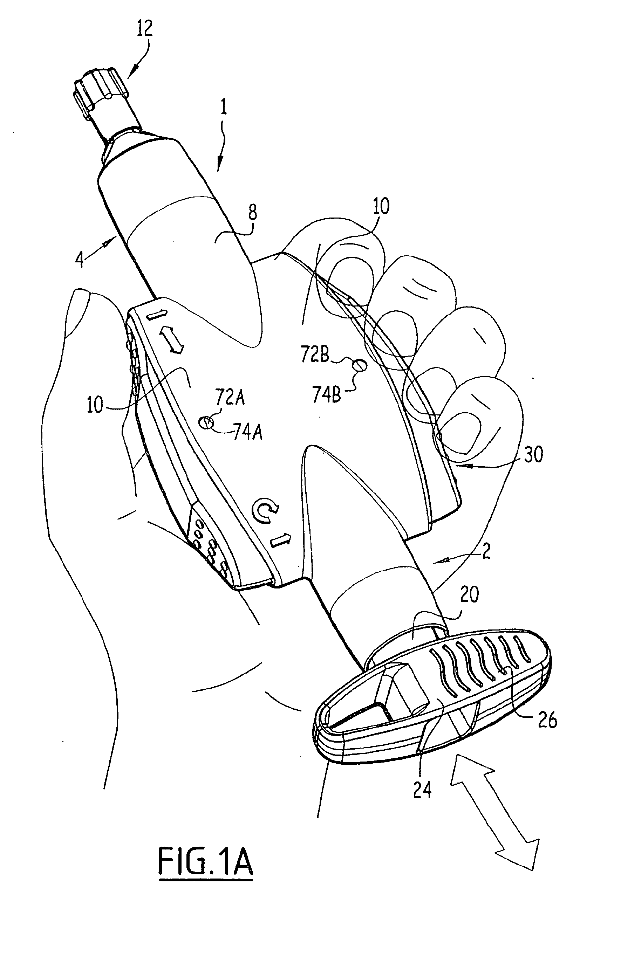

Le dispositif 1 de gonflage de ballonnet représenté sur les figures 1A, 1B, 2, 3A et 3B comporte une seringue 2 s'étendant le long d'un axe X-X et comprenant essentiellement un corps de seringue 4 et un piston 6. La seringue a avantageusement une contenance de 30 cm3.

Le corps de seringue 4, réalisé en matière plastique transparente, comporte une partie tubulaire 8 sur laquelle sont rapportés, de façon diamétralement opposée, deux boîtiers latéraux 10 venus de matière avec la partie tubulaire.

L'extrémité avant du corps de seringue, c'est-à-dire son extrémité de plus petit diamètre, est pourvu d'un ensemble de fixation 12 adapté pour recevoir, de manière amovible, un connecteur de forme complémentaire non représenté. Cet ensemble de fixation, couramment appelé « rotating », comporte un organe déformable 14 de retenue du connecteur et des moyens d'étanchéité 16 formés par exemple d'un joint.

Le piston de seringue 6 est formé d'une tige 20 munie à son extrémité reçue à l'intérieur de la partie tubulaire 8, au travers de l'extrémité arrière du corps de seringue 4, d'une tête 22 qui coulisse de manière étanche à l'intérieur du corps de seringue 4. La tige comporte, à son autre extrémité, une poignée 24 destinée à l'actionnement manuel du piston 6. Cette poignée est pourvue d'un revêtement agrippant 26 pour faciliter sa prise en main.

La tige 20 est pourvue extérieurement d'un filetage 28 sur au moins une partie de sa longueur.

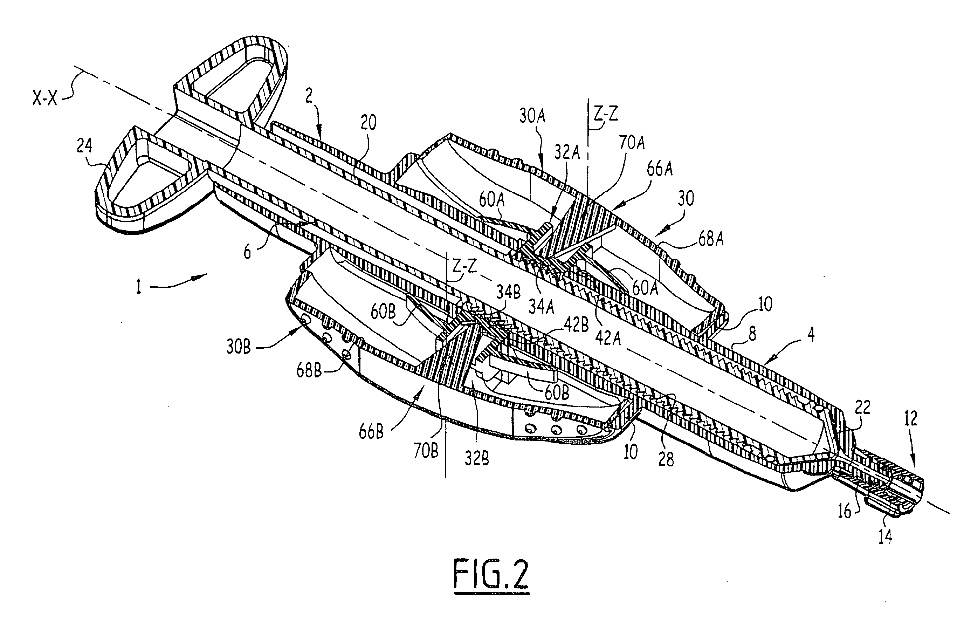

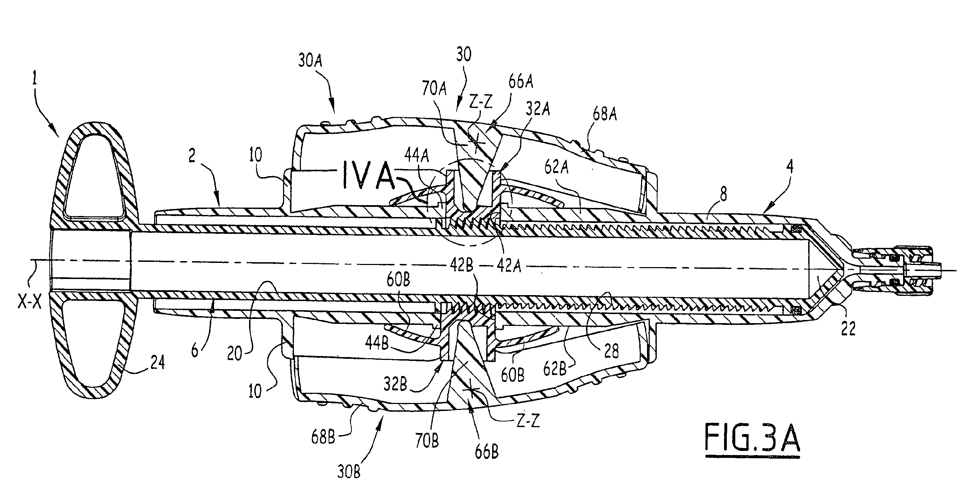

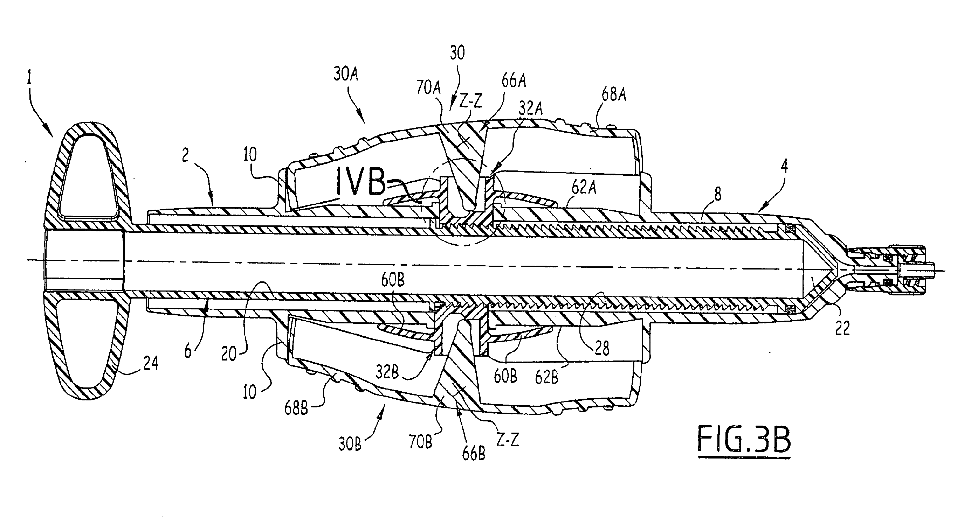

Le dispositif 1 comprend également un mécanisme 30 de retenue du piston 6, reçu pour l'essentiel à l'intérieur des boîtiers latéraux 10. Comme représenté sur les figures 2, 3A et 3B, ce mécanisme est formé de deux ensembles identiques 30A et 30B, reçus chacun dans un boîtier latéral, de manière symétrique par rapport à un plan médian perpendiculaire au plan de coupe de la figure 2. Par la suite, ne sera décrit en détail que l'ensemble 30A, étant entendu que l'ensemble 30B comporte les mêmes éléments que l'ensemble 30A et que les éléments identiques des ensembles portent la même référence numérique suivie de la lettre A pour l'ensemble 30A et de la lettre B pour l'ensemble 30B.

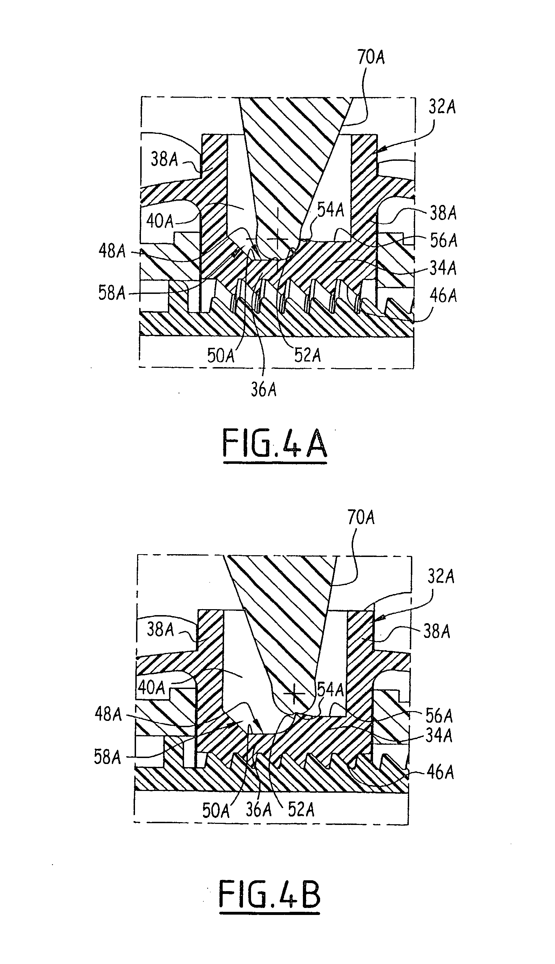

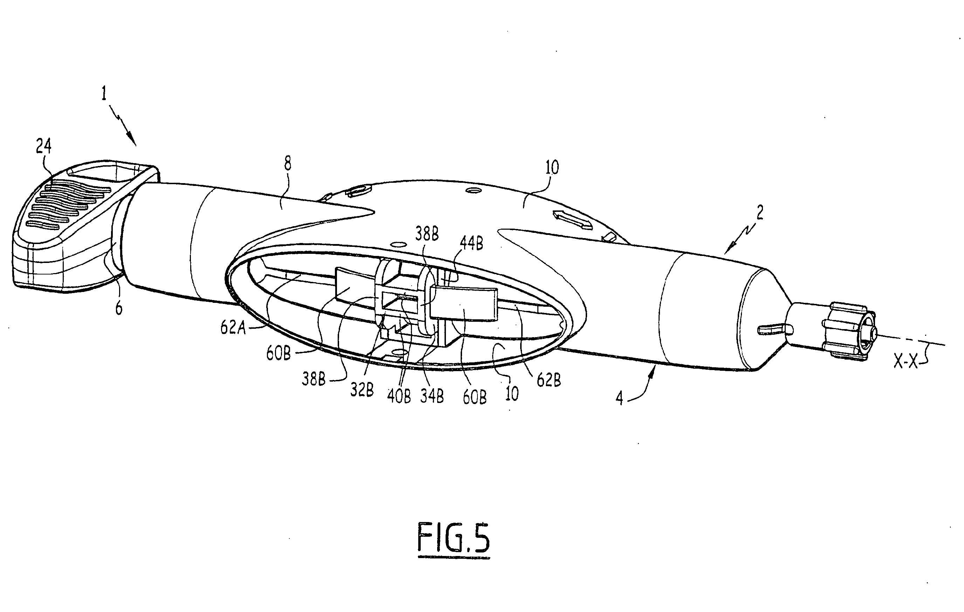

L'ensemble de retenue 30A comporte une demi-noix 32A comprenant un corps creux 34A, représenté plus en détail sur les figures 4A, 4B et 5. Ce corps est essentiellement formé d'une paroi de fond 36A qui s'étend parallèlement au piston de seringue 6, deux parois latérales opposées 38A qui s'étendent sensiblement perpendiculairement à l'axe X-X de la seringue, et deux parois latérales opposées 40A qui relient les parois 38A, une seule de ces parois 40A étant visible sur les figures 2, 3A, 3B, 4A et 4B.

La demi-noix 32A est reçue à l'intérieur d'une ouverture oblongue traversante 42A ménagée dans la partie tubulaire 8 du corps de seringue 4, et y est maintenue par une console de logement 44A solidaire du corps de seringue 4, par exemple venue de matière avec la partie tubulaire 8 et les boîtiers 10. Cette console 44A est nervurée de façon, d'une part, à autoriser le déplacement de la demi-noix suivant une direction sensiblement perpendiculaire à l'axe X-X et contenue dans le plan de coupe de la figure 2, et, d'autre part, bloquer la demi-noix à la fois suivant les autres directions perpendiculaires à l'axe X-X et parallèlement à cet axe.

La paroi de fond 36A de la demi-noix 32A présente, du côté dirigé vers l'intérieur du corps de seringue 4, une surface taraudée 46A destinée à venir en prise avec le filetage 28 du piston 6.

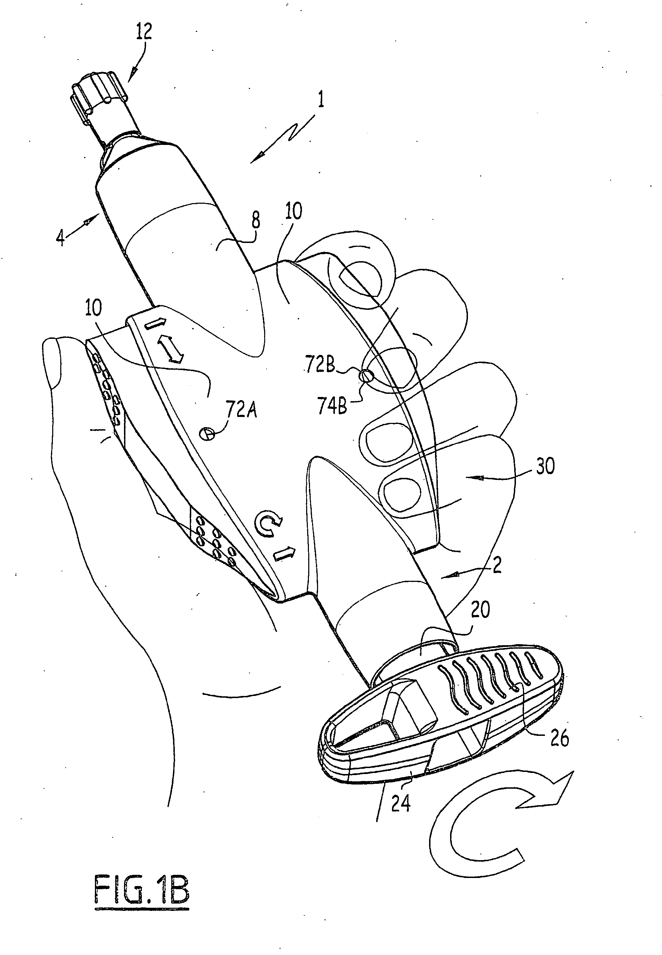

La demi-noix est ainsi déplaçable entre une position écartée du filetage 28, représentée sur les figures 1A et 3A, dans laquelle le piston 6 est libre de coulisser dans le corps de seringue 4, et une position en prise avec ce filetage, représentée sur les figures 1B et 3B, dans laquelle le piston 6 peut être vissé ou dévissé dans le corps de seringue 4, son libre coulissement étant impossible.

La paroi de fond 36A de la demi-noix 32A présente également, du côté dirigé vers l'extérieur du corps de seringue 4, une face étagée 48A, représentée en détail sur les figures 4A et 4B. Cette face comporte successivement, d'arrière en avant, une première surface sensiblement plane 50A, une surface inclinée vers l'extérieur formant rampe 52A, un col 54A de forme arrondie en saillie vers l'extérieur, et, en retrait vers l'intérieur par rapport au sommet du col 54A, une deuxième surface sensiblement plane 56A plus éloignée de l'axe de seringue X-X que la première surface 50A. Sur les figures 4A et 4B, les proportions respectives des surfaces 50A, 52A et 56A et du col 54A sont exagérées pour plus de visibilité.

L'ensemble de retenue 30A comporte également deux pattes déformables élastiquement 60A qui s'étendent de part et d'autre, suivant l'axe X-X, de la demi-noix 32A. Ces pattes 60A sont venues de matière avec les parois latérales 38A du corps 34A de la demi-noix. Les extrémités libres de ces pattes sont élastiquement en appui sur une nervure longitudinale 62A formée le long de la partie tubulaire 8 du corps de seringue.

L'ensemble de retenue 30A comporte en outre, comme représenté sur les figures 2, 3A et 3B, un organe 66A de commande du déplacement de la demi-noix 32A, entièrement réalisé en matière plastique, issue de moulage. Cet organe est formé d'une demi-coquille 68A dont l'évidement interne est tourné vers le corps de seringue 6, et d'un doigt 70A de forme générale pyramidale dont la base est venue de matière avec la demi-coquille.

La demi-coquille 68A forme un capot de fermeture du boîtier 10 correspondant, en étant mobile par rapport à ce dernier. Plus précisément, comme représenté sur les figures 1A et 1B, deux pions cylindriques 72A (un seul est visible sur les figures 1A et 1B), d'axe Z-Z perpendiculaire au plan de coupe des figure 2, 3A et 3B, sont formés en saillie du doigt 70A, chaque pion s'étendant depuis un des deux côtés du doigt 70A sensiblement parallèles au plan de coupe de la figure 2. Ces pions sont reçus dans des ouvertures 74A sensiblement complémentaires ménagées dans la paroi du boîtier 10. De la sorte, l'organe de commande 66A est basculable autour de l'axe Z-Z par rapport au corps de seringue 4.

Le doigt 70A est adaptée pour être reçue à l'intérieur du corps creux 34A de la demi-noix 32A et s'appuyer sur la face étagée 48A de cette demi-noix. Plus précisément, lorsque l'organe de commande 66A est basculé vers l'avant, comme représenté sur les figures 1A, 3A et 4A, l'extrémité libre du doigt 70A est en appui contre la surface 50A, les pattes élastiques 60A maintenant la demi-noix en position écartée, de sorte que le piston 6 est libre de coulisser. Lorsque l'organe 66A est basculé vers l'arrière, comme représenté sur les figures 1B, 3B et 4B, le doigt 70A est en appui contre la surface 56A et les pattes élastiques 60A sont déformées, de sorte que la demi-noix 32A est en prise avec le filetage 28 du piston pour n'autoriser que le vissage ou le dévissage de celui-ci.

Les déplacements du doigt sont guidés, et éventuellement limités, par les faces internes des parois latérales 38A et 40A du corps creux 34A. A titre d'exemple, pour le dispositif représenté, l'angle de basculement autour de l'axe Z-Z formé par le doigt est de l'ordre de 12°.

Le fonctionnement du dispositif de gonflage 1 est le suivant :

En vue du gonflage d'un ballonnet, l'opérateur relie, par exemple par une tubulure pourvue à son extrémité amont d'un connecteur, l'extrémité avant du dispositif 1 à un ballonnet, par fixation du connecteur sur l'ensemble 12.

Il saisit ensuite le dispositif comme représenté sur la figure 1A, c'est-à-dire en pressant manuellement, notamment entre son pouce et son index, la partie avant des demi-coquilles 68A et 68B, de façon à faire basculer vers l'avant les organes de commande 66A et 66B. L'opérateur peut alors pousser à l'intérieur du corps de seringue 4 la tige de piston 6 afin de faire augmenter la pression du fluide contenu dans la seringue, jusqu'à à une pression de l'ordre de 3 bars, ce qui correspond généralement à la pression pouvant être obtenue par simple poussée du piston.

Après cela, l'opérateur bascule vers l'arrière les organes de commande 66A et 66B, comme représenté sur la figure 1B, en exerçant une pression manuelle sur la partie arrière des demi-coquilles, notamment en pressant ces demi-coquilles entre sa paume et son auriculaire. Le basculement vers l'arrière des organes de commande provoque le déplacement des extrémités des doigts 70A, 70B depuis les surfaces 50A, 50B jusqu'aux surfaces 56A, 56B. Ces extrémités s'appuient lors de leur déplacement sur les rampes 52A et 52B, en provoquant, par effet de came, la mise en prise du filet 28 par les demi-noix 32A et 32B.

L'opérateur poursuit ensuite la montée en pression en vissant progressivement le piston dans le corps de seringue jusqu'à une pression pouvant atteindre, par exemple, 30 bars. Les demi-noix 32A et 32B sont fermement maintenues en prise avec le filetage du piston par les doigts 70A et 70B, les cols 54A et 54B des faces d'appui 48A, 48B des demi-noix assurant le blocage des organes de commande en position basculée vers l'arrière.

La libération des demi-noix s'effectue par la suite en répétant en sens inverse les étapes décrites ci-dessus.

Dans la seringue décrite ici, la présence des deux demi-noix 32A et 32B disposées symétriquement évite tout fléchissement de la tige 20 du piston 6.

De plus, l'engagement et le désengagement des demi-noix avec le filet 28 du piston 6 sont provoqués par le basculement des organes de commande 66A et 66B au moyen d'une prise en main ergonomique du dispositif. En effet, contrairement aux dispositifs de l'art antérieur, ces mouvements de basculement sont obtenus par serrage manuel d'une partie du dispositif disposé au creux d'une main de l'utilisateur, ce dernier développant une force plus importante que celle obtenue par le déplacement, par exemple, de son seul pouce.

Par ailleurs, le nombre de pièces constituant le dispositif est réduit, les boîtiers latéraux de réception des ensembles de retenue 30A, 30B étant formés d'un seul tenant avec le corps de seringue. Les coûts de fabrication et d'assemblage du dispositif s'en trouvent réduits.

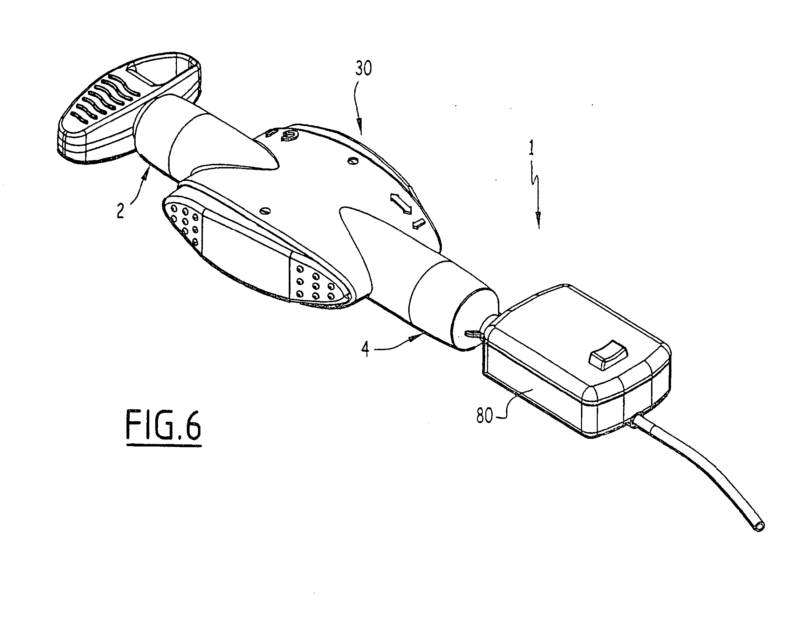

De plus, l'ensemble de fixation 12 porté par l'extrémité avant du dispositif 1 permet la mise en place d'une tubulure avec mesure de pression déportée, et/ou d'un manomètre 80, ce dernier pouvant tout aussi bien être de type mécanique à aiguille ou de type électronique avec capteur de pression, comme représenté sur la figure 6.

Le manomètre étant amovible, celui-ci est réutilisable. De plus, les différents types de manomètres peuvent être utilisés avec un même type de dispositif de gonflage de ballonnets.

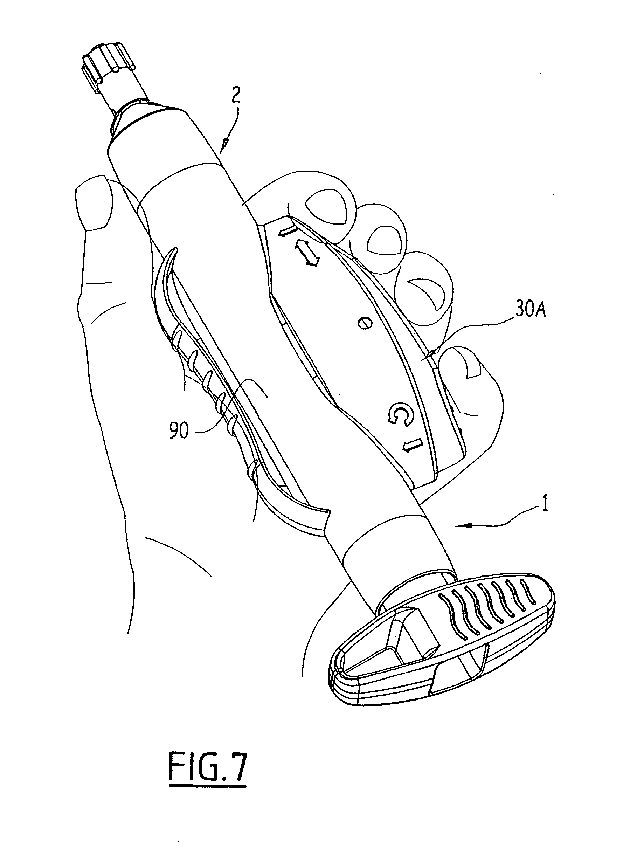

Sur la figure 7 est représentée une variante du dispositif 1 qui se distingue du dispositif des figures précédentes par le fait que son mécanisme de retenue du piston de seringue n'est formé que du seul ensemble 30A. Du côté du corps de seringue 4 opposé à cet ensemble 30A, le dispositif comporte une poignée 90 de forme ergonomique, avantageusement intégrée au corps de seringue 4.

Le fonctionnement de cette variante est identique à celui décrit ci-dessus.

D'autres aménagements et variantes aux dispositifs décrits plus haut sont envisageables :
- des plaquettes rigides s'étendent perpendiculairement aux parois latérales 38A, 38B et les pattes élastiques 60A, 60B sont remplacées par des butées élastiques, par exemple des ressorts, disposées entre les extrémités libres de chacune de ces plaquettes et la partie tubulaire 8 du corps de seringue 4 ; et/ou
- le profil des faces étagées 48A, 48B d'appui pour les doigts 70A, 70B présente des creux et/ou des bosses adaptés pour faciliter le blocage et le déblocage des extrémités de ces doigts lors des sollicitations en basculement des organes de commande.

## Revendications

1. Dispositif de gonflage de ballonnet, du type comportant une seringue (2) comprenant un corps de seringue (4) et un piston de seringue (6) déplaçable à coulissement et à rotation dans ledit corps de seringue (4), le piston (6) présentant un filetage extérieur (28) sur au moins une partie de sa longueur, le dispositif (1) comportant en outre un mécanisme (30) de retenue du piston (6) comprenant, d'une part, au moins une demi-noix (32A, 32B) mobile entre une position écartée du filetage (28) dans laquelle le piston (6) est libre de coulisser dans le corps de seringue (4), et une position en prise avec le filetage (28) dans laquelle le libre coulissement du piston (6) est impossible et dans laquelle ce piston peut être vissé ou dévissé dans le corps de seringue (4), et, d'autre part, pour chaque demi-noix (32A, 32B), un organe (66A, 66B) de commande du déplacement de la demi-noix correspondante entre ses deux positions, monté mobile par rapport au corps de seringue (4), dans lequel le mécanisme de retenue (30) comporte, pour chaque demi-noix (32A, 32B), au moins un élément déformable élastiquement (60A, 60B), en appui sur la demi-noix correspondante et sur le corps de seringue (4), et en ce que l'organe de commande (66A, 668) comporte un doigt (70A, 70B) d'appui sur la demi-noix correspondante (32A, 32B), adapté pour s'appuyer, lors du déplacement de l'organe de commande, contre deux surfaces (50A, 50B, 56A, 568) portées par la demi-noix et décalées l'une par rapport à l'autre suivant une direction radiale au corps de seringue (4), la demi-noix étant dans sa position écartée du filetage lorsque le doigt est en appui contre la surface (50A, 50B) la plus proche radialement du piston de seringue (6) et la demi-noix étant dans sa position en prise avec le filetage lorsque le doigt est en appui contre la surface (56A, 56B) la plus éloignée, **caractérisé en ce que** les deux surfaces (50A, 50B, 56A, 56B) portées par la demi-noix (32A, 32B) sont disposées l'une derrière l'autre suivant la direction longitudinale (X-X) du corps de seringue (4).

2. Dispositif suivant la revendication 1, **caractérisé en ce que** l'élément déformable (60A, 60B) est solidaire de la demi-noix correspondante (32A, 32B).

3. Dispositif suivant la revendication 1 ou 2, **caractérisé en ce que** l'élément déformable est une patte élastique (60A, 60B) qui s'étend sensiblement parallèlement à la direction longitudinale (X-X) du corps de seringue (4).

4. Dispositif suivant l'une quelconque des revendications précédentes, **caractérisé en ce que**, pour la ou chaque demi-noix (32A, 32B), sont prévus deux éléments déformables (60A, 60B) disposés de part et d'autre, suivant la direction longitudinale (X-X) du corps de seringue (4), de la demi-noix.

5. Dispositif suivant l'une quelconque des revendications précédentes, **caractérisé en ce que** la ou chaque demi-noix (32A, 32B) présente une surface (52A, 52B) de passage entre les deux surfaces (50A, 508, 56A, 56B) contre lesquelles s'appuie le doigt correspondant (70A, 70B), ladite surface de passage formant came pour ledit doigt.

6. Dispositif suivant l'une quelconque des revendications précédentes, **caractérisé en ce que** ladite surface la plus éloignée (56A, 56B) est pourvue d'une saillie (54) adaptée pour bloquer le doigt (70A, 70B) en appui contre ladite surface la plus éloignée.

7. Dispositif suivant l'une quelconque des revendications précédentes, **caractérisé en ce que** l'organe de commande (66A, 66B) de la ou chaque demi-noix (32A, 32B) est monté à basculement autour d'un axe (Z-Z) perpendiculaire à la direction longitudinale (X-X) du corps de seringue (4).

8. Dispositif suivant l'une quelconque des revendications précédentes, **caractérisé en ce que** le ou chaque organe de commande (66A, 66B) est reçu dans un boîtier (10) solidaire du corps de seringue (4) et sur lequel est monté mobile ledit organe de commande.

9. Dispositif suivant l'une quelconque des revendications précédentes, **caractérisé en ce que** la ou chaque demi-noix (32A, 32B) comporte un corps creux (34A, 34B) de réception d'une partie du doigt correspondant (70A, 70B), lequel corps creux comporte une paroi de fond (36A, 36B) portant lesdites deux surfaces (50A, 50B, 56A, 56B) contre lesquelles le doigt s'appuie, et des parois latérales (38A, 38B, 40A, 40B) formant des surfaces de guidage du doigt lors de ses déplacements par rapport au corps de seringue (4).

10. Dispositif suivant l'une quelconque des revendications précédentes, **caractérisé en ce que** le mécanisme (30) de retenue du piston de seringue (6) ne comporte qu'une seule demi-noix (30A), et **en ce qu'**il comporte une poignée rigide (90) venue de matière avec le corps de seringue (4) et située de manière diamétralement opposée à l'organe de commande (66A) de l'unique demi-noix (30A).

## Claims

1. Balloon inflation device, of a type comprising a syringe (2) having a syringe body (4) and a syringe plunger (6) which is slidably and rotatably displaceable in said syringe body (4), the plunger (6) having an external thread (28) over at least part of its length, the device (1) additionally comprising a mechanism (30) for retaining the plunger (6) comprising, firstly, at least one half-nut (32A, 32B) movable between a position spaced apart from the thread (28), in which the plunger (6) is free to slide within the syringe body (4) and a position in engagement with the thread (28), in which the free sliding of the plunger (6) is impossible and in which this plunger can be screwed or unscrewed in the syringe body (4), and, secondly, for each half-nut (32A, 32B), a member (66A, 66B) for control of the displacement of the corresponding half-nut between its two positions, which member is mounted so as to be movable relative to the syringe body (4), wherein the retaining mechanism (30) comprises, for each half-nut (32A, 32B), at least one resiliently deformable element (60A, 60B) bearing against the corresponding half-nut and against the syringe body (4), and the control member (66A, 66B) includes a finger (70A, 70B) bearing against the corresponding half-nut (32A, 32B), which finger is arranged, in the course of displacement of the control member, to bear against two surfaces (50A, 50B, 56A, 56B) located on the half-nut and offset from one another in a direction radial to the syringe body (4), the half-nut being in its position spaced apart from the thread when the finger is bearing against the surface (50A, 50B) that is radially closer to the syringe plunger (6) and the half-nut being in its position in engagement with the thread when the finger is bearing against the more remote surface (56A, 56B), **characterised in that** the two surfaces (50A, 50B, 56A, 56B) located on the half-nut (32A, 32B) are arranged one behind the other in the longitudinal direction (X-X) of the syringe body (4).

2. Device according to claim 1, **characterised in that** the deformable element (60A, 60B) is fixed to the corresponding half-nut (32A, 32B).

3. Device according to claim 1 or 2, **characterised in that** the deformable element is a resilient tab (60A, 60B) which extends substantially parallel to the longitudinal direction (X-X) of the syringe body (4).

4. Device according to any one of the preceding claims, **characterised in that**, for the or each half-nut (32A, 32B), there are provided two deformable elements (60A, 60B) arranged to one side and the other of the half-nut in the longitudinal direction (X-X) of the syringe body (4).

5. Device according to any one of the preceding claims, **characterised in that** the or each half-nut (32A, 32B) has a surface (52A, 52B) of transition between the two surfaces (50A, 50B, 56A, 56B) against which the corresponding finger (70A, 70B) bears, said transition surface forming a cam for said finger.

6. Device according to any one of the preceding claims, **characterised in that** said more remote surface (56A, 56B) is provided with a projection (54) arranged to block the finger (70A, 70B) bearing against said more remote surface.

7. Device according to any one of the preceding claims, **characterised in that** the control member (66A, 66B) of the or each half-nut (32A, 32B) is mounted so as to tilt about an axis (Z-Z) perpendicular to the longitudinal direction (X-X) of the syringe body (4).

8. Device according to any one of the preceding claims, **characterised in that** the or each control member (66A, 66B) is received in a housing (10) fixed to the syringe body (4) and in which said control member is movably mounted.

9. Device according to any one of the preceding claims, **characterised in that** the or each half-nut (32A, 32B) includes a recessed body (34A, 34B) for receiving part of the corresponding finger (70A, 70B), which recessed body includes a bottom wall (36A, 36B) on which there are located said two surfaces (50A, 50B, 56A, 56B) against which the finger bears, and side walls (38A, 38B, 40A, 40B) forming surfaces for guiding the finger in the course of its displacements relative to the syringe body (4).

10. Device according to any one of the preceding claims, **characterised in that** the mechanism (30) for retaining the syringe plunger (6) includes only one half-nut (30A) and **in that** it includes a rigid handle (90) integrally formed with the syringe body (4) and located diametrically opposite the control member (66A) of the single half-nut (30A).

## Patentansprüche

1. Ballonaufblasgerät umfassend:
eine Spritze (2) mit einem Spritzenkörper (4) und einem Spritzenkolben (6), der gleitend und drehend im Spritzenkörper (4) verstellbar ist, wobei der Kolben (6) ein Außengewinde (28) auf mindestens einem Teil seiner Länge aufweist;
wobei das Gerät außerdem eine Vorrichtung (30) zum Feststellen des Kolbens (6) aufweist,
wobei die Vorrichtung (30) zum Feststellen des Kolbens einerseits mindestens eine Halbnuss (32A, 32B) aufweist, die zwischen einer vom Gewinde (28) entfernten Stellung, in der der Kolben (6) frei im Spritzenkörper (4) gleiten kann, und einer in das Gewinde eingreifenden Stellung beweglich ist, in der das freie Gleiten des Kolbens unmöglich ist und in der dieser Kolben in den Spritzenkörper (4) hinein- oder herausgeschraubt werden kann; und
wobei die Vorrichtung (30) zum Feststellen des Kolbens andererseits für jede Halbnuss (32A, 32B) ein Bedienelement (66A, 66B) für das Verstellen der jeweiligen Halbnuss zwischen ihren zwei Stellungen aufweist,
wobei das Bedienelement (66A, 66B) beweglich in Bezug auf den Spritzenkörper (4) angebracht ist,
wobei die Vorrichtung zum Feststellen (30) für jede Halbnuss (32A, 32B) mindestens ein elastisch verformbares Element (60A, 60B) aufweist, das auf die jeweilige Halbnuss und auf den Spritzenkörper (4) drückt,
wobei das Bedienelement (66A, 66B) einen Finger (70A, 70B) zum Drücken auf die jeweilige Halbnuss (32A, 32B) aufweist, der dazu geeignet ist, beim Verstellen des Bedienelements (66A, 66B) gegen zwei Oberflächen (50A, 50B, 56A, 56B) der Halbnuss zu drücken, die entlang einer radialen Richtung im Spritzenkörper (4) gegeneinander versetzt sind,
wobei sich die Halbnuss in ihrer vom Gewinde entfernten Stellung befindet, wenn der Finger gegen die Oberfläche (50A, 50B) drückt, die radial dem Spritzenkolben (6) am nächsten ist, und wobei sich die Halbnuss in ihrer in das Gewinde eingerasteten Position befindet, wenn der Finger gegen die am weitesten entfernte Oberfläche (56A, 56B) drückt,
**dadurch gekennzeichnet, dass**
die zwei Oberflächen (50A, 50B, 56A, 56B) der Halbnuss (32A, 32B) hintereinander entlang der Längsrichtung (X-X) des Spritzenkörpers (4) angeordnet sind.

2. Gerät nach Anspruch 1, **dadurch gekennzeichnet, dass** das verformbare Element (60A, 60B) einstückig mit der jeweiligen Halbnuss (32A, 32B) ausgebildet ist.

3. Gerät nach Anspruch 1 oder 2, **dadurch gekennzeichnet, dass** das verformbare Element eine elastische Lasche (60A, 60B) ist, die sich im Wesentlichen parallel zur Längsrichtung (X-X) des Spritzenkörpers erstreckt.

4. Gerät nach einem der vorigen Ansprüche, **dadurch gekennzeichnet, dass** für die oder für jede Halbnuss (32A, 32B) zwei verformbare Elemente (60A, 60B) vorgesehen sind, die beiderseits von der Halbnuss (32A, 32B) entlang der Längsrichtung (X-X) des Spritzenkörpers (4) angeordnet sind.

5. Gerät nach einem der vorigen Ansprüche, **dadurch gekennzeichnet, dass** die oder dass jede Halbnuss (32A, 32B) eine Umstelloberfläche (52A, 52B) zwischen den zwei Oberflächen (50A, 50B, 56A, 56B) aufweist, auf die der jeweilige Finger (70A, 70B) drückt, wobei die Umstelloberfläche einen Nocken für den Finger darstellt.

6. Gerät nach einem der vorigen Ansprüche, **dadurch gekennzeichnet, dass** die am weitesten entfernte Oberfläche (56A, 56B) mit einem Vorsprung versehen ist, der dazu geeignet ist, den gegen die am weitesten entfernten Oberfläche drückenden Finger (70A, 70B) zu blockieren.

7. Gerät nach einem der vorigen Ansprüche, **dadurch gekennzeichnet, dass** das Bedienelement (66A, 66B) von der oder von jeder Halbnuss (32A, 32B) kippbar um eine Achse (Z-Z) senkrecht zur Längsrichtung (X-X) des Spritzenkörpers (4) angebracht ist.

8. Gerät nach einem der vorigen Ansprüche, **dadurch gekennzeichnet, dass** der oder dass jedes Bedienelement (66A, 66B) in einem Gehäuse (10) aufgenommen ist, das einstückig mit dem Spritzenkörper (4) ausgebildet ist und auf dem das Bedienelement beweglich angebracht ist.

9. Gerät nach einem der vorigen Ansprüche, **dadurch gekennzeichnet, dass** die oder dass jede Halbnuss (32A, 32B) einen Hohlkörper (34A, 34B) zum Aufnehmen eines Teils des jeweiligen Fingers (70A, 70B) aufweist, wobei der Hohlkörper eine Bodenwand (36A, 36B) mit den zwei Oberflächen (50A, 50B, 56A, 56B), gegen die der Finger drückt, und seitliche Wände (38A, 38B, 40A, 40B) aufweist, die Führungsflächen des Fingers beim Verstellen desselben in Bezug auf den Spritzenkörper (4) bilden.

10. Gerät nach einem der vorigen Ansprüche, **dadurch gekennzeichnet, dass** die Vorrichtung (30) zum Feststellen des Spritzenkolbens (6) nur eine einzige Halbnuss (30A) umfasst, und **dadurch**, dass das Gerät einen starren Griff (90) aufweist, der zusammen mit dem Spritzenköper (4) hergestellt ist und derart angeordnet ist, dass er diametral entgegengesetzt zum Bedienelement (66A) der einzigen Halbnuss (30A) ist.
